# EUROPEAN PATENT SPECIFICATION

(11) **EP 3 882 339 B1**
(45) Date of publication and mention of the grant of the patent: **22.01.2025**
(21) Application number: 19885228.7
(22) Date of filing: 11.11.2019
(51) Int. Cl.: C12N 5/071, C12N 5/077, C12N 5/0775, A61K 35/28, A61K 35/33

(54) **METHOD FOR PRODUCING INSULIN-PRODUCING CELLS**
VERFAHREN ZUR HERSTELLUNG VON INSULINPRODUZIERENDEN ZELLEN
PROCÉDÉ DE PRODUCTION DE CELLULES PRODUISANT DE L'INSULINE

(30) Priority: 14.11.2018 JP 2018213448
(43) Date of publication of application: 22.09.2021
(73) Proprietor: Kataoka Corporation, Kyoto-shi, Kyoto 601-8203 (JP); Kyoto Prefectural Public University Corporation, Kyoto-shi, Kyoto 602-8566 (JP)
(72) Inventor: DAI, Ping, Kyoto-shi, Kyoto 602-8566 (JP); TAKEDA, Yukimasa, Kyoto-shi, Kyoto 602-8566 (JP); HARADA, Yoshinori, Kyoto-shi, Kyoto 602-8566 (JP); MATSUMOTO, Junichi, Kyoto-shi, Kyoto 601-8203 (JP); KUSAKA, Ayumi, Kyoto-shi, Kyoto 601-8203 (JP)
(74) Representative: Vossius & Partner Patentanwälte Rechtsanwälte mbB
(86) International application number: PCT/JP2019/044058
(87) International publication number: WO 2020/100789

(56) References cited:
- EP-A1- 3 031 905
- WO-A1-2014/001771
- D. FOMINA-YADLIN ET AL: "Small-molecule inducers of insulin expression in pancreatic -cells", PROCEEDINGS OF THE NATIONAL ACADEMY OF SCIENCES, vol. 107, no. 34, 9 August 2010 (2010-08-09), pages 15099 - 15104, XP055200934, ISSN: 0027-8424, DOI: 10.1073/pnas.1010018107
- FOMINA-YADLIN DINA A: ""Small molecule inducers of insulin expression in pancreatic alpha cells"", PHD DISSERTATION, 6 July 2011 (2011-07-06), pages 1 - 210, XP055848413, Retrieved from the Internet <URL:https://hollis.harvard.edu/primo-explore/fulldisplay?docid=01HVD_ALMA212155280430003941&context=L&vid=HVD2&lang=en_US&search_scope=everything&adaptor=Local%20Search%20Engine&tab=everything&query=any,contains,Fomina-Yadlin%20Dina&offset=0> [retrieved on 20211006]
- SAIYONG ZHU ET AL: "Human pancreatic beta-like cells converted from fibroblasts", NATURE COMMUNICATIONS, vol. 7, 6 January 2016 (2016-01-06), pages 10080, XP055430582, DOI: 10.1038/ncomms10080
- BHAWNA CHANDRAVANSHI ET AL: "Reprogramming mouse embryo fibroblasts to functional islets without genetic manipulation", JOURNAL OF CELLULAR PHYSIOLOGY, WILEY SUBSCRIPTION SERVICES, INC, US, vol. 233, no. 2, 11 August 2017 (2017-08-11), pages 1627 - 1637, XP071323042, ISSN: 0021-9541, DOI: 10.1002/JCP.26068
- CHOUDHARY, AMIT ET AL.: "Quantitative-Proteomic Comparison of Alpha and Beta Cells to Uncover Novel Targets for Lineage Reprogramming", PLOS ONE, vol. 9, no. 4, 2014, pages 1 - 10, XP055707997, Retrieved from the Internet <URL:https://journals.plos.org/plosone/article/file?id=10.1371/journal.pone.0095194&type=printable> [retrieved on 20191125]
- TANIOKA, TOSHIHIRO: "Molecular mechanisms of insulin resistance in beta cells", THE SHOWA UNIVERSITY JOURNAL OF PHARMACEUTICAL SCIENCES, vol. 2, no. 2, 1 January 2011 (2011-01-01), JP, pages 127 - 138, XP009528083, ISSN: 1884-7854
- HORI, YUICHI ET AL.: "Growth inhibitors promote differentiation of insulin-producing tissue from embryonic stem cells", PNAS, vol. 99, no. 25, 2002, pages 16105 - 16110, XP002392968, DOI: 10.1073/pnas.252618999
- MA, XIAOJIE ET AL.: "Chemical strategies for pancreatic beta cell differentiation, reprogramming, and regeneration", ACTA BIOCHIM BIOPHYS SIN, vol. 49, no. 4, 2017, pages 289 - 301, XP055647725, DOI: 10.1093/abbs/gmx008

## Description

### TECHNICAL FIELD

### (Cross-Reference to Related Applications)

This application claims the benefit of Japanese Patent Application No. 2018-213448, filed on November 14, 2018, with the Japanese Patent Office.

The present invention is classified to the technical field of regenerative medicine, or direct reprogramming from somatic cells. The present invention relates in the technical field to an in vitro process for directly producing insulin-producing cells from somatic cells by low molecular weight compounds, and to low molecular weight compound-induced insulin-producing cells (ciIPCs: chemical compound-induced insulin-producing cells) produced by such a process. The present invention further relates to the insulin-producing cell, and a composition which can be used for the process of producing the insulin-producing cell.

### BACKGROUND OF THE INVENTION

A method is known in which brown adipocytes, osteoblasts, cartilage cells, nervous system cells, or cardiomyocytes are directly induced from somatic cells, in particular human fibroblasts, without performing gene transfer, by culturing the somatic cells under a combination of several low molecular weight compounds such as an ALK5 inhibitor and an ALK6 inhibitor (e.g., Patent Document 1). As in the invention of Patent Document 1, it is very beneficial that conventional fibroblasts can be directly induced into cells such as brown adipocytes by a low molecular weight compound which is easy to obtain. For example, autologous fibroblasts can be conveniently used to produce other autologous cells, thereby increasing their applications in regenerative medicine. Moreover, cells can be easily produced as experimental materials for the development of new pharmaceuticals.

The cells of the mesenchymal system form various organs of the living body such as muscle, bone, cartilage, bone marrow, fat and connective tissue, and are promising as materials of regenerative medicine. Mesenchymal stem cells (MSCs) are undifferentiated cells present in tissues such as bone marrow, adipose tissue, blood, placenta and umbilical cord. Because of their ability to differentiate into cells belonging to the mesenchymal system, the mesenchymal stem cells have attracted attention as a starting material in the production of these cells. Regenerative medicine using the mesenchymal stem cells themselves for reconstruction of bone, cartilage, myocardium, or the like is also being investigated.

Actually, some examples of differentiation from mesenchymal stem cells into nerve cells and insulin-producing cells are also reported (Non-Patent Documents 1 and 2). In Non-Patent Document 1, mesenchymal stem cells are differentiated into nerve cells by a combination of low molecular weight inhibitors such as SB431542 and dorsomorphine.

For insulin-secreting pancreatic β-cells, in addition to the reports of differentiation induction from iPS-cells or ES-cells to human pancreatic β-cells, it has been reported that endodermal cells such as pancreatic α-cells, pancreatic acinar cells, pancreatic ductal glandular cells, small intestinal crypt cells, hepatocytes, and cholangiocytes were directly induced to pancreatic β-cells using Pdx1, Ngn,3, and MafA, which are pancreatic β-cell-specific transcription factors (Non-patent Document 3). Moreover, direct induction from mouse embryonic fibroblasts (MEFs) or human dermal fibroblasts to pancreatic β-cells using Yamanaka-4-factors has been reported (Non-Patent Documents 4 and 5). In addition, it has been reported that endoderm progenitor cells were produced from mouse embryonic fibroblasts (MEFs) by low molecular weight compounds and differentiated into pancreatic endocrine cells (Non-Patent Document 6).

BRD7389, a low molecular weight compound that is an RSK inhibitor, is reported to have a capacity of activating the expression of insulin genes in pancreatic α-cells (Non-Patent Document 7).
Fomina-Yadlin Dina A: ""Small molecule inducers of insulin expression in pancreatic alpha cells"", PhD Dissertation, 6 July 2011 (2011-07-06), pages 1-210 corresponds to Non-Patent Document 7 and i.a. discloses that that BRD7389 is a small-molecule activator of insulin expression in alpha cells and the effect of p53 activation by GW8510 on beta cell differentiation.
WO 2014/001771 A1 discloses a method for reprogramming a somatic cell (pancreatic ductal cell, a skin fibroblast or a keratinocyte) to a pancreatic beta-cell.
EP 3 031 905 A1 discloses the use of forskolin and/or CHIR99021 for generating pancreatic hormone-producing cells from progenitor cells.
BHAWNA CHANDRAVANSHI ET AL; JOURNAL OF CELLULAR PHYSIOLOGY, vol. 233, no. 2, 11 August 2017 (2017-08-11), pages 1627-1637 describes reprogramming mouse embryo fibroblasts to functional islets without genetic manipulation.

### PRIOR ART DOCUMENTS

### PATENT DOCUMENT

Patent Document 1: WO 2018/062269

### NON-PATENT DOCUMENT

Non-Patent Document 1: Stem Cells International, Volume 2016, Article ID 1035374
Non-Patent Document 2: BioMed Research International, Volume 2015, Article ID 575837
Non-Patent Document 3: Current Pathobiology Reports, 2016, Vol. 3, pp. 57-65
Non-Patent Document 4: Cell Stem Cell, 2014, Vol. 14, pp. 228-236
Non-Patent Document 5: Nature Communications, 2016, Vol. 7, p. 10080
Non-Patent Document 6: Journal of Biological Chemistry, 2017, Vol. 292, pp. 19122-19132
Non-Patent Document 7: PNAS, August 24 (2010), Vol. 107, no. 34, pp. 15099-15104

### SUMMARY OF THE INVENTION

### PROBLEM TO BE SOLVED BY THE INVENTION

Like the process described in Patent Document 1, a method of performing direct conversion from somatic cells to desired cells without performing gene transfer is an effective option as a means of obtaining therapeutic cells. As described above, methods for direct conversion from somatic cells have also been reported for pancreatic β-cells, but in these inventions the conversions have been induced by introducing specific genes into endodermal cells that share a close developmental lineage.

The present invention is primarily directed to providing a new in vitro process of efficiently and directly inducing the differentiation to an insulin-producing cell from a somatic cell by a combination of low molecular weight compounds without performing artificial gene transfer, namely, a new in vitro process capable of directly producing an insulin-producing cell from a somatic cell by a certain low molecular weight compound.

### MEANS FOR SOLVING THE PROBLEMS

As a result of intensive studies, the present inventors have found that a somatic cell can be efficiently and directly converted into an insulin-producing cell by culturing the somatic cell in the presence of a certain low molecular weight inhibitor or the like, and the present invention was completed.

The present invention includes the following as claimed in appended claims 1 to 6:
[1.] An in vitro process for producing an insulin-producing cell by direct differentiation induction from a somatic cell selected from the group consisting of a fibroblast and a mesenchymal stem cell as a starting material, comprising a step of culturing the somatic cell in a differentiation induction medium in the presence of an RSK inhibitor, a cAMP inducer and a GSK3 inhibitor, wherein the RSK inhibitor is BRD7389 or BI-D1870, the GSK3 inhibitor is CHIR99021 and the cAMP inducer is forskolin and wherein the differentiation induction medium does not contain serum.
[2.] The in vitro process for producing an insulin-producing cell according to [1.] , wherein the somatic cell is cultured in the further presence of the PI3K inhibitor LY294002.
[3.] The in vitro process for producing an insulin-producing cell according to [1.] or [2.], wherein the somatic cell is a fibroblast.
[4.] Use of a composition for producing an insulin-producing cell by directly inducing differentiation from a somatic cell selected from the group consisting of a fibroblast and a mesenchymal stem cell as a starting material in a differentiation induction medium, wherein the composition comprises an RSK inhibitor, a cAMP inducer and a GSK3 inhibitor, wherein the RSK inhibitor is BRD7389 or BI-D1870, the cAMP inducer is forskolin and the GSK3 inhibitor is CHIR99021, wherein the differentiation induction medium does not contain serum.
[5.] Use according to [4.], wherein the composition further comprises the PI3K inhibitor LY294002.
[6.] Use according to [4.] or [5.], wherein the somatic cell is a fibroblast.

### EFFECT OF THE INVENTION

According to the present invention, insulin-producing cells having a high secretion ability can be efficiently and directly produced from somatic cells without performing artificial gene transfer. The insulin-producing cells obtained according to the present invention are useful in regenerative medicine and the like.

### BRIEF DESCRIPTION OF THE DRAWINGS

Figure 1 represents the amount of insulin secreted by insulin-producing cells directly differentiated from human fibroblasts. The vertical axis indicates insulin secretion per mg of total protein of the cell (µU/mg).
Figure 2 represents the amount of insulin secreted by insulin-producing cells directly differentiated from human fibroblasts. The vertical axis indicates insulin secretion per mg of total protein of the cell (µU/mg).
Figure 3 represents the amount of insulin secreted by insulin-producing cells directly differentiated from human fibroblasts. The vertical axis indicates insulin secretion per mg of total protein of the cell (µU/mg).
Figure 4 represents the amount of insulin secreted by insulin-producing cells directly differentiated from human fibroblasts. The vertical axis indicates insulin secretion per mg of total protein of the cell (µU/mg).
Figure 5 represents the amount of insulin secreted by insulin-producing cells directly differentiated from human adipose tissue-derived mesenchymal stem cells (AdMSC). The vertical axis indicates insulin secretion per mg of total protein of the cell (µU/mg).
Figure 6 represents the amount of insulin secreted by insulin-producing cells directly differentiated from human adipose tissue-derived mesenchymal stem cells (AdMSC). The vertical axis indicates insulin secretion per mg of total protein of the cell (µU/mg).

### EMBODIMENTS FOR CARRYING OUT THE PRESENT INVENTION

Hereinafter, the present invention will be described in detail.

### 1 Process for producing insulin-producing cells

The in vitro process for producing an insulin-producing cell according to the present invention (hereinafter, referred to as "the present invention process") is an in vitro process for producing an insulin-producing cell by direct differentiation induction from a somatic cell, wherein the differentiation medium does not contain serum.

Moreover, the present invention process comprises a step of culturing a somatic cell in the presence of an RSK inhibitor, a GSK3 inhibitor, and in the presence of a cAMP inducer. The above step may be a step of culturing a somatic cell optionally in the presence of other inhibitors, inducers, or the like, as required.

### 1.1 Somatic cells

Cells of an organism can be classified into somatic and germ cells. Any somatic cell can be used as a starting material in the present invention process. A somatic cell is not particularly limited, and may be either a primary cell taken from a living body or a cell from an established cell line. Somatic cells at various stages of differentiation, e.g., terminally differentiated somatic cells (e.g., fibroblasts, umbilical vein endothelial cells (HUVEC), liver cells (Hepatocytes), bile duct cells (Biliary cells), pancreatic alpha cells (Pancreatic α cells), pancreatic acinar cells (Acinar cells), pancreatic duct gland cells (Ductal cells), small intestinal crypt cells (Intestinal crypt cells), and the like), somatic cells on the way to terminal differentiation (e.g., mesenchymal stem cells, neural stem cells, endodermal progenitor cells, and the like), or somatic cells that have been reprogrammed and acquired pluripotency, can be used in the present invention process. Somatic cells include any somatic cells, for example, cells of the hematopoietic system (various lymphocytes, macrophages, dendritic cells, bone marrow cells, and the like), cells derived from organs (hepatocytes, splenocytes, pancreatic cells, kidney cells, lung cells, and the like), cells of the muscle tissue system (skeletal muscle cells, smooth muscle cells, myoblasts, cardiomyocytes, and the like), fibroblasts, neurons, osteoblasts, chondrocytes, endothelial cells, interstitial cells, adipocytes (white adipocytes, and the like), embryonic stem cells (ES-cells), and the like. In accordance with the present invention process, fibroblasts or mesenchymal stem cells are used.

The fibroblasts that can be used in the present invention are not particularly limited, and can include, for example, dermal fibroblasts, adventitial fibroblasts, cardiac fibroblasts, pulmonary fibroblasts, uterine fibroblasts, and villous mesenchymal fibroblasts, which are major cell components forming connective tissues in various tissues and organs, and produce collagen fibers.

The mesenchymal stem cells that can be used in the present invention are not particularly limited, and can include, for example, adipose tissue-derived mesenchymal stem cells, bone marrow-derived mesenchymal stem cells, umbilical cord-derived mesenchymal stem cells, umbilical cord blood-derived mesenchymal stem cells, dental pulp-derived mesenchymal stem cells, placenta-derived mesenchymal stem cells, amniotic membrane-derived mesenchymal stem cells, endometrium-derived mesenchymal stem cells, synovium-derived mesenchymal stem cells, dermal tissue-derived mesenchymal stem cells, and periodontal ligament-derived mesenchymal stem cells.

Examples of the source of the above-mentioned somatic cells include, but are not limited to, humans, mammals other than humans, and animals other than mammals (birds, reptiles, amphibians, fish, and the like). As a source of somatic cells, humans and mammals other than humans are preferred, and humans are particularly preferred. When insulin-producing cells are produced by the present invention process for the purpose of administration to humans, preferably, somatic cells harvested from a donor having a matched or similar type of histocompatibility antigen to the recipient can be used. Somatic cells harvested from the recipient itself may be used for the production of insulin-producing cells.

### 1.2 Inhibitors and the like according to the present invention

### 1.2.1 RSK inhibitors

RSK (Ribosomal S6 Protein Kinase) is widely expressed in cells and is one of the serine-threonine kinases that respond to various growth factors. It is classified into subfamilies with molecular weights of 70 kDa and 90 kDa. In particular, the 90 kDa RSK subfamily is activated by phosphorylation by ERK belonging to MAPK signaling pathway, and is derived from four genes in mammals. Activated 90 kDa RSK phosphorylate a variety of down-stream proteins, including Ribosomal protein S6, and regulate cell viability, cell proliferation, and differentiation in a variety of ways.

The term "in the presence of an RSK inhibitor" refers to a culture condition capable of inhibiting RSK, and the means thereof is not particularly limited, and substances that inhibit the activity of RSK, for example, RSK signal inhibiting means such as an anti-RSK antibody or an RSK inhibitor, can be used. Moreover, since RSK is activated when its own specific site is phosphorylated, the means of inhibiting the above-mentioned phosphorylation can also be used to inhibit the RSK signal.

The RSK inhibitor in the present invention is BRD7389 or BI-D1870.

### BRD7389 (CAS No.: 376382-11-5)

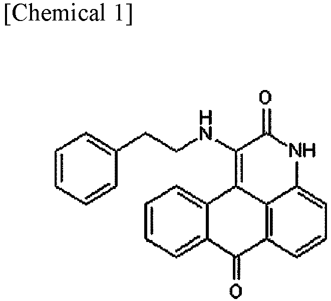

SL 0101-1 (CAS No.: 77307-50-7)*
BI-D1870 (CAS No.: 501437-28-1)
LJH685 (CAS No.: 1627710-50-2)*
LJI308 (CAS No.: 1627709-94-7)*
FMK (CAS No.: 821794-92-7)*
RMM46 (CAS No.: 1307896-46-3)*
CMK (CAS No.: 821794-90-5)*
Carnosol (CAS No.: 5957-80-2)*
Bix 02565 (CAS No.: 1311367-27-7)*
(* RSK inhibitors not included in the present invention)

The concentration of the RSK inhibitor varies depending on a somatic cell or the like to be used, but is not particularly limited, and may be appropriately determined, and can be used, for example, within the range of 0.05 µmol/L to 50 µmol/L, and preferably within the range of 0.1 µmol/L to 20 µmol/L.

### 1.2.2 GSK3 inhibitors

GSK3 (glycogen synthase kinase-3) was found as a protein kinase that phosphorylates and inactivates glycogen synthase. In mammals, GSK3 is classified into two isoforms: 51 kDa α (GSK3α) and 47 kDa β (GSK3β). GSK3 has the activity of phosphorylating various proteins and is involved not only in glycogen metabolism but also in physiological phenomena such as cell division and cell growth.

The term "in the presence of a GSK3 inhibitor" refers to a culture condition capable of inhibiting GSK3, and the means thereof is not particularly limited, and substances that inhibit the activity of GSK3, for example, GSK3 signal inhibiting means such as an anti-GSK3 antibody and a GSK3 inhibitor, can be used. Moreover, since GSK3 loses its activity when its own specific site is phosphorylated, the means of promoting the above-mentioned phosphorylation can also be used to inhibit the GSK3 signal.

The GSK3 inhibitor in the present invention is CHIR99021.

### CHIR99021 (CAS No.: 252917-06-9)

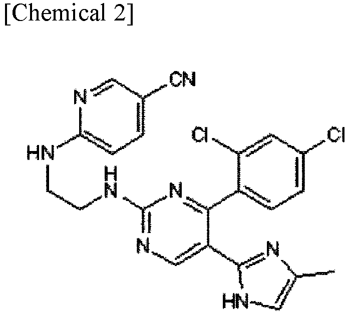

BIO ((2'Z,3'E)-6-Bromoindirubin-3'-oxime) (CAS No.: 667463-62-9)*
Kenpaullone (CAS No.: 142273-20-9)*
A1070722 (CAS No.: 1384424-80-9)*
SB216763 (CAS No.: 280744-09-4)*
CHIR98014 (CAS No.: 556813-39-9)*
TWS119 (CAS No.: 601514-19-6)*
Tideglusib (CAS No.: 865854-05-3)*
SB415286 (CAS No.: 264218-23-7)*
Bikinin (CAS No.: 188011-69-0)*
IM-12 (CAS No.: 1129669-05-1)*
1-Azakenpaullone (CAS No.: 676596-65-9)*
LY2090314 (CAS No.: 603288-22-8)*
AZD1080 (CAS No.: 612487-72-6)*
AZD2858 (CAS No.: 486424-20-8)*
AR-A014418 (CAS No.: 487021-52-3)*
TDZD-8 (CAS No.: 327036-89-5)*
Indirubin (CAS No.: 479-41-4)*
(* GSK3 inhibitors not included in the present invention)

The concentration of the GSK3 inhibitor varies depending on a somatic cell and the like to be used, but is not particularly limited, and may be appropriately determined, and can be used, for example, within the range of 0.05 µmol/L to 20 µmol/L, and preferably within the range of 0.1 µmol/L to 10 µmol/L.

### 1.2.3 cAMP inducers

cAMP (cyclic adenosine monophosphate) is a second messenger that is involved in a variety of intracellular signaling events. cAMP is produced intracellularly by cyclization of adenosine triphosphate (ATP) by adenylyl cyclase.

The term "in the presence of a cAMP inducer" refers to a culture condition capable of inducing cAMP, and the means thereof is not particularly limited, and for example, any means capable of increasing the intracellular cAMP concentrations can be used. Substances that can induce cAMP by acting directly on adenylyl cyclase, an enzyme involved in the production of cAMP, substances that can promote the expression of adenylyl cyclase, and substances that inhibit phosphodiesterase, an enzyme that degrades cAMP, can be used as means to increase intracellular cAMP concentrations. It is also possible to use dibutyryl cAMP, a structural analogue of cAMP, which has the same effect as cAMP in cells.

The cAMP inducer (adenylate cyclase activator) in the present invention is forskolin (CAS No.: 66575-29-9).

forskolin (CAS No.: 66428-89-5) 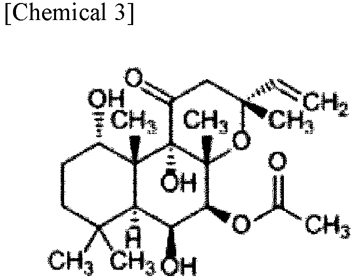
Isoproterenol (CAS No.: 7683-59-2)*
NKH477 (CAS No.: 138605-00-2)*
PACAP1-27 (CAS No.: 127317-03-7)*
PACAP1-38 (CAS No.: 137061-48-4)*
(* cAMP inducers not included in the present invention)

The concentration of the cAMP inducer varies depending on a somatic cell and the like to be used, but is not particularly limited, and may be appropriately determined, and can be used, for example, within the range of 0.2 µmol/L to 50 µmol/L, and preferably within the range of 1 µmol/L to 30 µmol/L.

### 1.2.4 PI3K inhibitors

PI3K (Phosphoinositide 3-kinase) is an enzyme that phosphorylates an inositol phospholipid, and a phosphoinositide produced activates PDK1. The PDK1 further phosphorylates AKT and activates PDK1/AKT signaling pathway. LY294002 is a selective inhibitor of PI3K and inhibits activation of PDK1/AKT signaling pathway by suppressing the production of phosphoinositide.

The term "in the presence of a PI3K inhibitor" refers to a culture condition capable of inhibiting PI3K, and the means thereof is not particularly limited, and any means capable of inhibiting PI3K can be used. In the present invention, substances that act directly on PI3K to inhibit its function (e.g., anti-PI3K antibodies, and other agents), agents that inhibit the production of PI3K themselves, and the like can be used. PI3K can also be inhibited by inhibiting the upstream signaling pathway of PI3K.

The PI3K inhibitor in the present invention is LY294002.

### LY294002 (CAS No.: 154447-36-6)

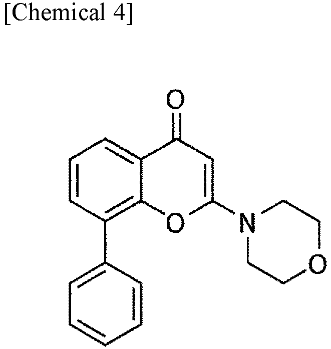

Buparlisib (CAS No.: 944396-07-0)*
TGR-1202 (CAS No.: 1532533-67-7*)
PI-103 (CAS No.: 371935-74-9)*
IC-87114 (CAS No.: 371242-69-2)*
Wortmannin (CAS No.: 19545-26-7*)
ZSTK474 (CAS No.: 475110-96-4)*
AS-605240 (CAS No.: 648450-29-7)*
PIK-90 (CAS No.: 677338-12-4)*
AZD6482 (CAS No.: 1173900-33-8)*
Duvelisib (CAS No.: 1201438-56-3)*
TG100-115 (CAS No.: 677297-51-7)*
CH5132799 (CAS No.: 1007207-67-1)*
CAY10505 (CAS No.: 1218777-13-9)*
PIK-293 (CAS No.: 900185-01-5)*
CZC24832 (CAS No.: 1159824-67-5)*
Pilaralisib (CAS No.: 934526-89-3)*
AZD8835 (CAS No.: 1620576-64-8)*
(* PI3K inhibitors not included in the present invention)

The concentration of the PI3K inhibitor varies depending on a somatic cell and the like to be used, but is not particularly limited, and may be appropriately determined, and can be used, for example, within the range of 0.1 µmol/L to 20 µmol/L, and preferably within the range of 0.5 µmol/L to 10 µmol/L.

### 1.3 Somatic cell culture

Culturing of somatic cells in the present invention process can be carried out in the presence of various inhibitors described above (and, optionally, inducers or activators), by selecting a medium, a temperature, or other conditions depending on the type of somatic cells to be used.

In the present invention process, by culturing a somatic cell in a differentiation induction medium containing various inhibitors and the like described above, an insulin-producing cell can be produced from a somatic cell by one step culture.

Moreover, by selecting a somatic cell for use which can be easily cultured, it is possible to increase the number of cells in advance and convert the somatic cell which has reached a substantially confluent state into an insulin-producing cell. Thus, a scaled-up production of insulin-producing cells is also easy.

The differentiation induction medium or the subculture medium for a somatic cell, which are the bases for carrying out the present invention, can be selected from known or commercially available media. For example, MEM (Minimum Essential Medium), DMEM (Dulbecco's Modified Eagle's Medium), or DMEM/F12, which are a common medium, or a modified medium thereof, can be used by adding appropriate components (proteins, amino acids, saccharides, vitamins, fatty acids, antibiotics, and the like) thereto.

The culture of somatic cells in the present invention process is performed without containing serum such as fetal bovine serum (FBS) in a differentiation induction medium. Moreover, it is preferable that the culture is carried out in a medium containing a large amount of insulin. In addition, it is more preferable that the differentiation induction medium does not contain serum such as fetal bovine serum (FBS) and contains a large amount of insulin.

In one embodiment of the present invention process, it is preferable to culture a somatic cell by containing a large amount of insulin in a differentiation induction medium, and the amount thereof may be, for example, 5 µg/mL or more, preferably 20 µg/mL or more, or 25 µg/mL or more, and more preferably within the range of 80 µg/mL to 120 µg/mL.

When a certain amount of insulin is previously contained in the known or commercially available basal induction medium, insulin may be added thereto to adjust the insulin content to the above amount.

As a culture condition, a general condition of cell culture can be selected. Conditions of 37°C and 5% CO₂, and the like are exemplified. It is preferred to change the medium at appropriate intervals during culture (preferably once every one to five days, and more preferably once every two to four days). When the present invention process is carried out using fibroblasts as materials, insulin-producing cells appear in between six to eight days and 12 days under conditions of 37°C and 5% CO₂. When the present invention process is carried out using mesenchymal stem cells as materials, insulin-producing cells appear in about one week under the conditions of 37°C and 5% CO₂.

For culturing somatic cells, a cell culture container such as a plate, a dish, a cell culture flask, a cell culture bag, or the like can be used. Note that, as the cell culture bag, one having gas permeability is suitable. A large culture vessel may be used when large amounts of cells are required. The culture can be carried out in either an open system or a closed system, but when the purpose is administration or the like of the obtained insulin-producing cells to a human, it is preferable to carry out the culture in a closed system.

### 1.4 Insulin-producing cells

By the present invention process described above, a cell population containing insulin-producing cells can be obtained. The insulin-producing cell produced by the present invention process is also within the scope of the present invention. The insulin-producing cell produced by the present invention process may be not only a terminally differentiated cell but also a progenitor cell destined to differentiate into an insulin-producing cell.

The insulin-producing cell produced by the present invention process is a so-called low molecular weight compound-induced insulin-producing cell (ciIPCs), which is induced to differentiate directly from a somatic cell by a low molecular weight compound, and is distinguished from that induced to differentiate by gene transfer.

The insulin-producing cell produced by the present invention process can be detected, identified, and isolated using, for example, a morphological change of a cell, a characteristic property of an insulin-producing cell, or a specific marker (e.g., an anti-insulin antibody). Moreover, the secretory capacity of the obtained insulin-producing cell can also be evaluated by quantifying the amount of secreted insulin using sandwich ELISA.

Quarantine methods (detection with an antibody) can be used to detect specific markers, and as for protein molecules, detection may be performed with quantitation of their mRNA quantities. An antibody that recognizes the specific marker of the insulin-producing cell is also useful for isolating and purifying the insulin-producing cell obtained by the present invention process.

The insulin-producing cell produced by the present invention process can be used, for example, in methods for tissue repair, improvement of blood insulin concentration, and the like. By transplanting the insulin-producing cell produced by the present invention process, a pharmaceutical composition for tissue repair or the like can be produced. Pancreas transplantation or islet transplantation has become a radical treatment for patients with type 1 diabetes, who are congenitally almost completely deficient in insulin secretion, to alleviate and cure their symptoms. Moreover, the number of patients with type 2 diabetes is expected to increase further in Japan and overseas, and cause medical costs to rise, but transplantation of pancreatic β-cells secreting insulin may be an effective therapy. As a means for treating pancreatic diseases such as diabetes, a process for producing insulin-producing cells and a method for transplanting insulin-producing cells have been developed. For example, transplantation of insulin-producing cells under the kidney capsule or transplantation into the liver via the portal vein is expected to be used in methods for the treatment of severe pancreatic diseases such as diabetes.

When the insulin-producing cell produced by the present invention process is used as a pharmaceutical composition, it may be prepared in a formulation suitable for administration to an individual, such as by mixing the insulin-producing cell with a pharmaceutically acceptable carrier, in a conventional method. Examples of the carrier can include physiological saline and distilled water for injection which is made isotonic by adding glucose or other adjuvants (e.g., D-sorbitol, D-mannitol, sodium chloride, and the like). Furthermore, buffering agents (e.g., a phosphate buffer, a sodium acetate buffer), painless agents (e.g., benzalkonium chloride, procaine hydrochloride, and the like), stabilizers (e.g., human serum albumin, polyethylene glycol, and the like), preservatives, antioxidants, and the like may be blended.

The insulin-producing cell produced by the present invention process may also be made into a composition further combined with other cells or components effective for improving the functional performance and the engraftment of the insulin-producing cell.

Furthermore, the insulin-producing cell produced by the present invention process can also be used for screening of a pharmaceutical candidate compound which acts on an insulin-producing cell and for evaluating the safety of a pharmaceutical candidate compound. Insulin-producing cells are an important tool for evaluating the toxicity of a pharmaceutical candidate compound. According to the present invention process, a large number of insulin-producing cells can be acquired in a single operation, and then it is possible to obtain reproducible research results without being affected by a lot difference in cells.

### 2 Compositions

The composition according to the present invention (hereinafter, referred to as "the present invention composition") referred to in appended use claims 4 to 6 is a composition for producing an insulin-producing cell from a somatic cell by directly inducing differentiation.

The present invention provides the use of said composition for producing insulin-producing cells from somatic cells and as a medium for producing insulin-producing cells from somatic cells.

As the medium used for producing insulin-producing cells from somatic cells is a medium containing an RSK inhibitor as an active ingredient, the GSK3 inhibitor and the cAMP inducer and as required, the PI3K inhibitor in a basal medium produced by mixing components necessary for culturing cells. The active ingredient described above may be contained in a concentration effective for producing an insulin-producing cell, and the concentration may be appropriately determined by those skilled in the art. The basal medium can be selected from known or commercially available media. For example, MEM (Minimum Essential Medium), DMEM (Dulbecco's Modified Eagle's Medium), DMEM/F12, or RPMI1640, which are a common medium, or a medium modified therewith, can be used as the basal medium.

The medium according to the present invention composition does not contain serum such as fetal bovine serum (FBS). It is also preferable that it contains a large amount of insulin. In addition, it is more preferable that it does not contain serum such as fetal bovine serum (FBS) and contains a large amount of insulin.

In one embodiment of the medium according to the present invention composition, it is preferable that the medium contains a large amount of insulin, and the amount thereof can be, for example, 5 µg/mL or more, preferably 20 µg/mL or more, or 25 µg/mL or more, and more preferably within the range of 80 µg/mL to 120 µg/mL.

When a certain amount of insulin is previously contained in the known or commercially available basal induction medium, insulin may be added to prepare the present invention composition so that insulin is contained in the above amount.

A known medium component described above herein, such as serum, proteins (albumin, transferrin, a growth factor, and the like), amino acids, saccharides, vitamins, fatty acids, antibiotics, and the like may be further added to the medium according to the present invention composition.

Substances effective for inducing differentiation into insulin-producing cells, described above, may further be added to the medium according to the present invention composition.

Furthermore, it is disclosed that insulin-producing cells can also be produced from somatic cells by administering, for example, RSK inhibitors, optionally further GSK3 inhibitors, and/or cAMP inducers or PI3K inhibitors to a living body. For example, by administering RSK inhibitors, optionally further GSK3 inhibitors, and/or cAMP inducers or PI3K inhibitors to a specific site in vivo, insulin-producing cells can be produced from somatic cells at the specific site in vivo. The above described is not part of the invention and not covered by the claims.

### EXAMPLES

Hereinafter, the present invention will be illustrated in more detail with reference to Examples.

### EXAMPLE 1: PRODUCTION OF INSULIN-PRODUCING CELLS

### - Direct induction from a human fibroblast to an insulin-producing cell -

### (1) Human fibroblasts

The human fibroblasts used as the material were purchased from DS Pharma Biomedical Co., Ltd. The fibroblasts are derived from 38-year-old human skin.

### (2) Direct induction from human fibroblasts into insulin-producing cells

Human fibroblasts were seeded in 35-mm dishes coated with gelatin (Cat#: 190-15805, manufactured by Wako Pure Chemical Industries, Ltd.) at 5 × 10⁴ cells/dish, and cultured in DMEM medium (manufactured by Gibco) supplemented with 10% fetal bovine serum (FBS), 100 U/mL penicillin, and 100 µg/mL streptomycin until confluent under conditions of 37°C and 5% CO₂. Note that DMEM refers to Dulbecco's Modified Eagle's Medium.

The medium of dishes of the human fibroblasts described above was replaced with the differentiation induction medium described below.
- Differentiation induction medium: Advanced DMEM/F-12 (Cat#: 12634010; manufactured by Gibco) supplemented with ITS-X (Cat#: 51500056, manufactured by Gibco), non-essential amino acids (NEAA; Cat#: 11140050, manufactured by Gibco), Glutamine (manufactured by Gibco; final concentration: 2 mmol/L), Nicotinamide (Cat#: 72340-100G, manufactured by Sigma-Aldrich; final concentration: 10 mmol/L), Exendin-4 (Cat#: av120214, manufactured by Abcam; final concentration: 100 ng/mL), 100 U/mL penicillin, 100 µg/mL streptomycin, insulin (Cat#: 093-06351; manufactured by Wako), and low molecular weight compounds described below, with or without addition of 10% fetal bovine serum (FBS, manufactured by Hyclone).

Subsequently, the cells were cultured under conditions of 37°C and 5% CO₂ while the medium was changed to the medium of the same composition every three days.

### <Low Molecular Weight Compounds>

3 µM CHIR99021 (Cat#: 13122, Cayman Chemical)
7.5 µM forskolin (Cat#: 063-02193, Wako)
1.5 µM BRD7389 (Cat#: ab146161, Abcam)
5 µM LY294002 (Cat#: 70920, Cayman Chemical)

### (3) Results

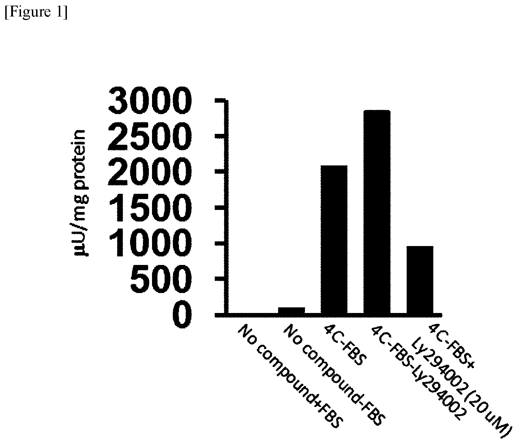

The results of culturing for 14 days according to (2) described above are shown in Figures 1 to 4.

In the Figures, the term "4C" refers to the above-mentioned four low molecular weight compounds, and the term "3C" refers to three compounds of the above-mentioned four low molecular weight compounds, which are CHIR99021, BRD7389, and forskolin. The term "+FBS" indicates the presence of FBS in the medium, and the term "-FBS" indicates the absence of FBS in the medium. The term "+LY294002 (20 µM)" indicates that LY294002 was present in the medium at a final concentration of 20 µM, and the term "-LY294002" indicates that no LY294002 was present in the medium (i.e., the term "4C-LY294002" has the same meaning as 3C). Moreover, the term "No Compound" indicates the absence in the medium of the above-mentioned four low molecular weight compounds. Thus, for example, the term "No Compound-FBS" represents the experimental results obtained when no 4C is present and no FBS is present, and the term "4C-FBS-LY294002" represents the experimental results obtained when LY294002 is absent from 4C and no FBS is present.

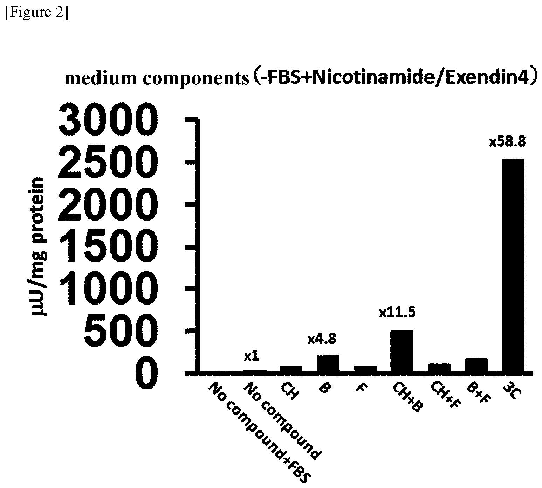

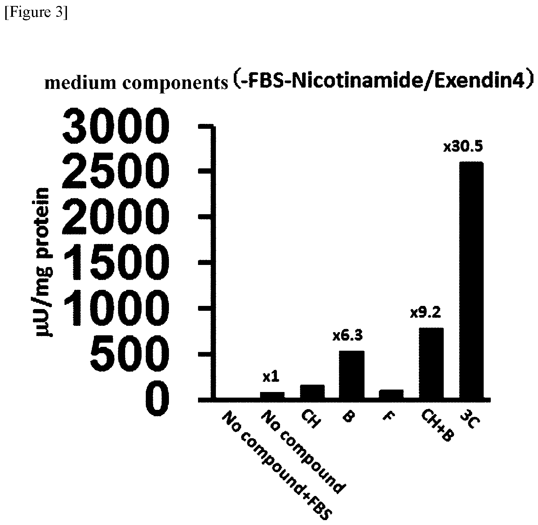

In Figures 2 and 3, the term "CH" refers to CHIR99021 and represents the experimental results obtained when only CHIR99021 of 4C or 3C is present. The term "B" refers to BRD7389 and represents the experimental results obtained when only BRD7389 of 4C or 3C is present. The term "F" refers to forskolin and represents the experimental results obtained when only forskolin of 4C or 3C is present. The term "+" as in the term "CH+B", for example, represents the experimental results obtained when both are present. The term "3C" represents the experimental results obtained when all compounds of 3C are present. The term "+Nicotinamide/Exendin4" indicates that nicotinamide and Exendin4 were added as medium components, and the term "-Nicotinamide/Exendin4" indicates that neither nicotinamide nor Exendin4 was added as medium components.

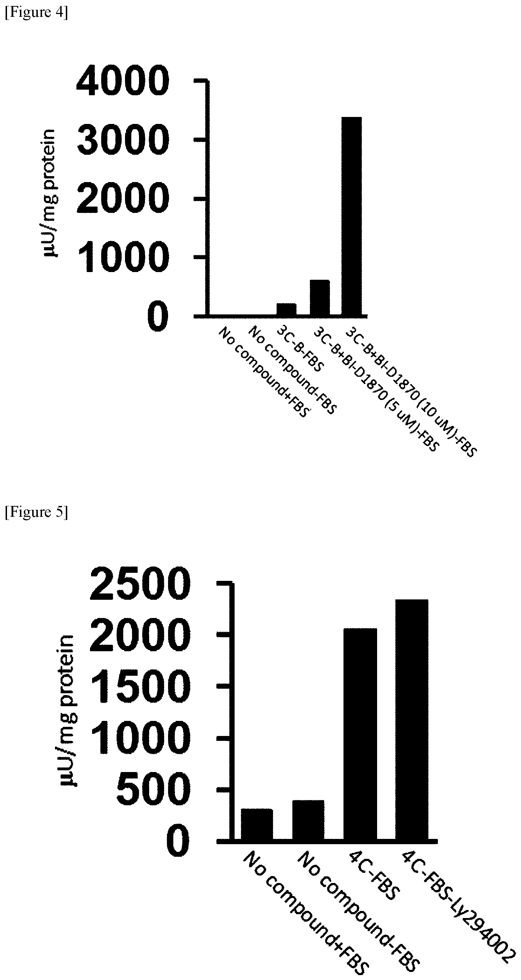

In Fig. 4, the term "B" refers to BRD7389, and the term "3C-B" represents the experimental results obtained in the absence of BRD7389 from 3C, namely, experimental results with two factors of CH and F. The term "3C-B+BI-D1870" represents the experimental results obtained when BRD7389 was absent among 3C, and instead BI-D1870 (Cat#: 15264, Cayman Chemical), an RSK inhibitor, was present at a final concentration of 5 µM or 10 µM.

As shown in Fig. 1, the presence of the above-mentioned low molecular weight compounds (an RSK inhibitor, a GSK inhibitor, a cAMP inducer, and a PI3K inhibitor) in the differentiation induction medium enables the direct induction of highly secretory insulin-producing cells from human fibroblasts efficiently, and among these, insulin-producing cells with a high secretory capacity were obtained even if the PI3K inhibitor (LY294002) was not present. As shown in Fig. 2 and Fig. 3, insulin secretion was synergistically increased by the combination of the three factors of the RSK inhibitor (BRD7389), the GSK inhibitor (CHIR99021), and the cAMP inducer (forskolin); however, a considerable amount of insulin secretion was observed by the combination of the two factors of the RSK inhibitor (BRD7389) and the GSK inhibitor (CHIR99021), or by only one factor of the RSK inhibitor (BRD7389), and induction to insulin-producing cells was confirmed. This tendency was observed as well when nicotinamide and Exendin-4 were not added in the medium (see Fig. 3).

From the results of Fig. 4, the two factors, in the absence of LY294002, and further in the absence of the RSK inhibitor (BRD7389) among the three factors consisting of the RSK inhibitor (BRD7389), the GSK inhibitor (CHIR99021), and the cAMP inducer (forskolin), led to a low level of insulin secretion. It is suggested that the presence of RSK inhibitors is important to the induction to insulin-producing cells. On the other hand, insulin-producing cells with a higher secretory capacity were also obtained by three factors in which BI-D1870, another RSK inhibitor, was present instead of BRD7389.

Incidentally, in case in which fetal bovine serum (FBS) was not contained in the differentiation induction medium, insulin-producing cells with a higher secretory capacity were obtained from human fibroblasts.

### EXAMPLE 2: PRODUCTION OF INSULIN-PRODUCING CELLS

### - Direct Induction from Human Mesenchymal Stem Cells to Insulin-Producing Cells -

### (1) Human mesenchymal stem cells

Human mesenchymal stem cells isolated from adipose tissue were purchased from Takara Bio Co., Ltd.

### (2) Direct Induction into Insulin-Producing Cells from Human Mesenchymal Stem Cells.

Human mesenchymal stem cells were seeded in 35 mm dishes coated with gelatin (Cat#: 190-15805, manufactured by Wako Pure Chemical Industries, Ltd.) at 5 × 10⁴ cells/dish, and cultured in Mesenchymal Stem Cell Growth Medium 2 (Cat#: C-28009; manufactured by Takara Bio Co., Ltd.) supplemented with 100 U/mL penicillin and 100 µg/mL streptomycin until the cells were almost confluent under conditions of 37°C and 5% CO₂.

The medium of dishes of the human mesenchymal stem cells described above was replaced with the differentiation induction medium described below.
- Differentiation induction medium: Advanced DMEM/F-12 (Cat#: 12634010; manufactured by Gibco) supplemented with ITS-X (Cat#: 51500056, manufactured by Gibco), non-essential amino acids (NEAA; Cat#: 11140050, manufactured by Gibco), Glutamine (manufactured by Gibco; final concentration: 2 mmol/L), Nicotinamide (Cat#: 72340-100G, manufactured by Sigma-Aldrich; final concentration: 5 mmol/L), Exendin-4 (Cat#: av120214, manufactured by Abcam; final concentration: 50 ng/mL), 100 U/mL penicillin, 100 µg/mL streptomycin, and low molecular weight compounds described below, with or without addition of 10% fetal bovine serum (FBS, manufactured by Hyclone).

Subsequently, the cells were cultured under conditions of 37°C and 5% CO₂ while the medium was changed to the medium of the same composition every three days.

Note that in experiments in which the induction medium contains 120 µg/mL of insulin, human recombinant insulin (Cat#: 093-06351; Wako) was added to adjust the medium to contain 120 µg/mL of insulin.

### <Low Molecular Weight Compounds>

0.5 µM CHIR99021 (Cat#: 13122, Cayman Chemical)
3.75 µM forskolin (Cat#: 063-02193, Wako)
0.2 µM BRD7389 (Cat#: ab146161, Abcam)
2.5 µM LY294002 (Cat#: 70920, Cayman Chemical)

### (3) Results

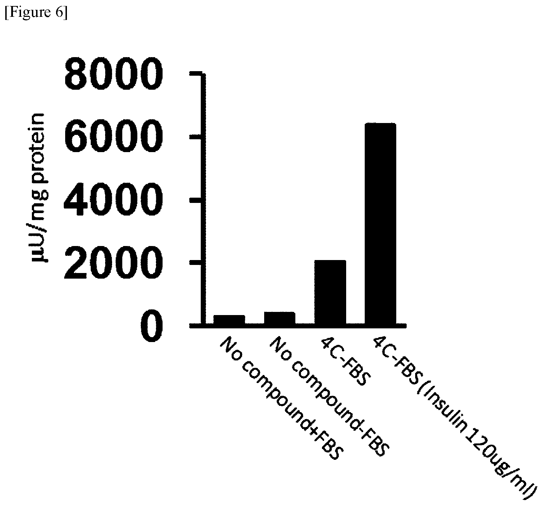

The results of culturing for 14 days according to the (2) described above are shown in Fig. 5 and Fig. 6. In the Figures, the term "4C" refers to the above-mentioned four low molecular weight compounds. The term "+FBS" indicates the presence of FBS in the medium, and the term "-FBS" indicates the absence of FBS in the medium. The term "-LY294002" indicates that no LY294002 was present in the medium (i.e., the term "4C-LY294002" has the same meaning as 3C). Moreover, the term "No compound" indicates the absence in the medium of the above-mentioned four low molecular weight compounds. Thus, for example, the term "No Compound-FBS" represents the experimental results obtained when no 4C is present and no FBS is present, and the term "4C-FBS-LY294002" represents the experimental results obtained when LY294002 is absent from 4C and no FBS is present.

As shown in Fig. 5 and Fig. 6, the presence of the above-mentioned low molecular weight compounds in the differentiation induction medium enabled efficient direct induction of highly secretory insulin-producing cells from human adipose tissue-derived mesenchymal stem cells (AdMSC). Insulin-producing cells with a high secretory capacity were obtained even in the absence of LY294002.

Moreover, in case in which fetal bovine serum (FBS) was not contained in the differentiation induction medium, insulin-producing cells with a higher secretory capacity were directly induced from human mesenchymal stem cells. Furthermore, with the induction by containing a high concentration (120 µg/mL) of insulin in the differentiation induction medium, the amount of insulin secretion increased significantly, and insulin-producing cells with a higher secretory capacity were efficiently induced directly from human mesenchymal stem cells.

## Claims

1. An in vitro process for producing an insulin-producing cell by direct differentiation induction from a somatic cell selected from the group consisting of a fibroblast and a mesenchymal stem cell as a starting material, comprising a step of culturing the somatic cell in a differentiation induction medium in the presence of an RSK inhibitor, a cAMP inducer and a GSK3 inhibitor, wherein the RSK inhibitor is BRD7389 or BI-D1870, the GSK3 inhibitor is CHIR99021 and the cAMP inducer is forskolin and wherein the differentiation induction medium does not contain serum.

2. The in vitro process for producing an insulin-producing cell according to Claim 1 , wherein the somatic cell is cultured in the further presence of the PI3K inhibitor LY294002.

3. The in vitro process for producing an insulin-producing cell according to claim 1 or 2, wherein the somatic cell is a fibroblast.

4. Use of a composition for producing an insulin-producing cell by directly inducing differentiation from a somatic cell selected from the group consisting of a fibroblast and a mesenchymal stem cell as a starting material in a differentiation induction medium, wherein the composition comprises an RSK inhibitor, a cAMP inducer and a GSK3 inhibitor, wherein the RSK inhibitor is BRD7389 or BI-D1870, the cAMP inducer is forskolin and the GSK3 inhibitor is CHIR99021, wherein the differentiation induction medium does not contain serum.

5. Use according to Claim 4, wherein the composition further comprises the PI3K inhibitor LY294002.

6. Use according to Claim 4 or 5, wherein the somatic cell is a fibroblast.

## Patentansprüche

1. *In vitro-*Verfahren zur Herstellung einer Insulin-produzierenden Zelle durch direkte Differenzierungsinduktion aus einer somatischen Zelle, die ausgewählt ist aus der Gruppe bestehend aus einem Fibroblasten und einer mesenchymalen Stammzelle als Ausgangsmaterial, umfassend einen Schritt des Kultivierens der somatischen Zelle in einem Differenzierungsinduktionsmedium in Gegenwart eines RSK-Inhibitors, eines cAMP-Induktors und eines GSK3-Inhibitors, wobei der RSK-Inhibitor BRD7389 oder BI-D1870 ist, der GSK3-Inhibitor CHIR99021 ist und der cAMP-Induktor Forskolin ist und wobei das Differenzierungsinduktionsmedium kein Serum enthält.

2. *In vitro*-Verfahren zur Herstellung einer Insulin-produzierenden Zelle nach Anspruch 1, wobei die somatische Zelle in der weiteren Gegenwart des PI3K-Inhibitors LY294002 kultiviert wird.

3. *In vitro*-Verfahren zur Herstellung einer Insulin-produzierenden Zelle nach Anspruch 1 oder 2, wobei die somatische Zelle ein Fibroblast ist.

4. Verwendung einer Zusammensetzung zur Herstellung einer Insulin-produzierenden Zelle durch direktes Induzieren der Differenzierung aus einer somatischen Zelle, die ausgewählt ist aus der Gruppe bestehend aus einem Fibroblasten und einer mesenchymalen Stammzelle als Ausgangsmaterial in einem Differenzierungsinduktionsmedium, wobei die Zusammensetzung einen RSK-Inhibitor, einen cAMP-Induktor und einen GSK3-Inhibitor umfasst, wobei der RSK-Inhibitor BRD7389 oder BI-D1870 ist, der cAMP-Induktor Forskolin ist und der GSK3-Inhibitor CHIR99021 ist, wobei das Differenzierungsinduktionsmedium kein Serum enthält.

5. Verwendung nach Anspruch 4, wobei die Zusammensetzung des Weiteren den PI3K-Inhibitor LY294002 umfasst.

6. Verwendung nach Anspruch 4 oder 5, wobei die somatische Zelle ein Fibroblast ist.

## Revendications

1. Procédé in vitro pour la production d'une cellule produisant de l'insuline par induction de différenciation directe à partir d'une cellule somatique choisie dans le groupe comprenant un fibroblaste et une cellule souche mésenchymateuse comme matériau de départ, comprenant une étape de culture de la cellule somatique dans un milieu d'induction de différenciation en présence d'un inhibiteur de RSK, d'un inducteur de cAMP et d'un inhibiteur de GSK3, dans lequel l'inhibiteur de RSK est BRD7389 ou BI-D1870, l'inhibiteur de GSK3 est CHIR99021 et l'inducteur de cAMP est la forskoline, et dans lequel le milieu d'induction de différenciation ne contient pas de sérum.

2. Procédé in vitro de production d'une cellule produisant de l'insuline selon la revendication 1, dans lequel la cellule somatique est cultivée en la présence supplémentaire de l'inhibiteur de PI3K LY294002.

3. Procédé in vitro de production d'une cellule produisant de l'insuline selon la revendication 1 ou 2, dans lequel la cellule somatique est un fibroblaste.

4. Utilisation d'une composition de production d'une cellule produisant de l'insuline en induisant directement une différenciation à partir d'une cellule somatique choisie dans le groupe comprenant un fibroblaste et une cellule souche mésenchymateuse en tant que matériau de départ dans un milieu d'induction de différenciation, dans laquelle la composition comprend un inhibiteur de RSK, un inducteur de cAMP et un inhibiteur de GSK3, dans laquelle l'inhibiteur de RSK est BRD7389 ou BI-D1870, l'inducteur de cAMP est la forskoline et l'inhibiteur de GSK3 est CHIR99021, dans laquelle le milieu d'induction de différenciation ne contient pas de sérum.

5. Utilisation selon la revendication 4, dans laquelle la composition comprend en outre l'inhibiteur de PI3K LY294002.

6. Utilisation selon la revendication 4 ou 5, dans laquelle la cellule somatique est un fibroblaste.
